# EUROPEAN PATENT APPLICATION

(11) **EP 4 834 287 A1**
(43) Date of publication of application: **04.11.2026**
(21) Application number: 24930404.9
(22) Date of filing: 10.10.2024
(51) Int. Cl.: A61F 2/24

(54) **HEART VALVE STENT AND HEART VALVE PROSTHESIS**

(30) Priority: 18.03.2024 CN 202410307689
(71) Applicant: Mitrassist Lifesciences Limited, Shanghai 201807 (CN)
(72) Inventor: LIANG, Xiangbin, Shanghai 201807 (CN); DUAN, Junhao, Shanghai 201807 (CN); ZHONG, Wei, Shanghai 201807 (CN)
(74) Representative: Patentanwälte Magenbauer & Kollegen Partnerschaft mbB
(86) International application number: PCT/CN2024/123975
(87) International publication number: WO 2025/194745

(57) **Abstract**

Provided are a heart valve stent (100) and a heart valve prosthesis (1000). The heart valve stent (100) comprises a stent body (10) and a first positioning part (20) and a second positioning part (30) used for positioning relative to heart tissues. The stent body (10) has defined therein a flow channel for blood to flow, and an upstream direction and a downstream direction are defined according to a direction (X) in which the blood flows through the flow channel. The first positioning part (20) and the second positioning part (30) are both located at the upstream end of the flow channel, and the first positioning part (20) and the second positioning part (30) both extend radially outward from the stent body (10), wherein the second positioning part (30) is located on the upstream side of the first positioning part (20); along the circumferential direction of the flow channel, the first positioning part (20) and the second positioning part (30) at least partially overlap, and along the axial direction of the flow channel, the first positioning part (20) is spaced apart from the second positioning part (30). The heart valve stent (100) exhibits high stability and good heart tissue anchoring performance.

## Description

### CROSS-REFERENCE TO THE RELATED APPLICATIONS

The present invention claims priority to Chinese Patent Application No. 202410307689.6, filed with the China National Intellectual Property Administration on March 18, 2024, and entitled "HEART VALVE STENT AND HEART VALVE PROSTHESIS", the contents of which are incorporated herein by reference in entirety.

### TECHNICAL FIELD

The present invention relates to the field of medical technology, and specifically, relates to a heart valve stent and a heart valve prosthesis.

### BACKGROUND

Heart valve is located between the atrium and the ventricle, and between the ventricle and the large artery, functions as one-way valve and is configured to guide unidirectional blood flow. The four valves of the human body are respectively the mitral valve, tricuspid valve, aortic valve and pulmonary valve. If lesions (such as stenosis or insufficiency) occur on these valves, blood flow will be affected, thereby causing abnormal cardiac function and ultimately leading to heart failure.

At present, valve replacement surgery is mostly adopted for treatment when valves develop lesions, namely replacement with artificial mechanical valves or bioprosthetic valves. However, the existing heart valve stents are prone to shedding under the influence of blood flow after implantation, featuring poor mounting and fixing stability, shortening the service life of artificial heart valves and increasing the risk of secondary valve replacement for patients.

### SUMMARY

Embodiments of the present invention provide a heart valve stent and a heart valve prosthesis with high mounting stability and strong anchoring performance for heart valves.

Embodiments of the present invention provide a heart valve stent. The heart valve stent includes a stent body, and a first positioning part and a second positioning part configured to position relative to cardiac tissues. The stent body defines a flow channel for blood circulation therein. An upstream direction and a downstream direction are defined according to the direction in which blood flows through the flow channel. Both the first positioning part and the second positioning part are located at an upstream end of the flow channel, and both the first positioning part and the second positioning part extend radially outward from the stent body, wherein the second positioning part is located on an upstream side of the first positioning part. Along a circumferential direction of the flow channel, the first positioning part and the second positioning part at least partially overlap. Along an axial direction of the flow channel, a spacing is provided between the first positioning part and the second positioning part.

In the present technical solution, a flow channel for blood circulation is defined in a middle portion of the stent body of the heart valve stent to allow blood to flow therethrough. Both the first positioning part and the second positioning part of the heart valve stent are disposed at the upstream end of the flow channel. The first positioning part and the second positioning part abut against and clamp two sides of corresponding cardiac tissues to fix the heart valve stent, preventing displacement of the heart valve stent caused by pressure exerted by blood on leaflets when the leaflets are closed. Therefore, the stability and reliability of the heart valve stent on cardiac tissues are improved, and the service life of the heart valve stent is prolonged. Moreover, the second positioning part is disposed on the upstream side of the first positioning part, that is, the second positioning part abuts against a ventricular side of cardiac tissues, and the first positioning part abuts against the other side of the cardiac tissues. The first positioning part and the second positioning part at least partially overlap, and a spacing is provided between the first positioning part and the second positioning part along the axial direction of the flow channel, such that the first positioning part and the second positioning part respectively clamp two opposite sides of the cardiac tissues with strong anchoring stability. The second positioning part has low rigidity and good compliance, and exerts little influence on conduction bundles.

In some embodiments, a circumferential extension angle of the first positioning part or the second positioning part is α, where α is greater than 320°.

In some embodiments, a connecting ring is disposed on the stent body, and the connecting ring is configured to be connected with a delivery system.

In the above embodiment, the connecting ring is formed on the stent body and connected with the delivery system, so that the heart valve stent can be delivered to a target position of cardiac tissues via the delivery system, improving the stability and reliability during delivery of the heart valve stent. In addition, arrangement of the connecting ring enables full retrieval of the heart valve stent.

In some embodiments, the first positioning part includes a plurality of first positioning units arranged along a circumferential direction. Each first positioning unit includes two first positioning rods. Two first positioning rods belonging to the same first positioning unit are connected at ends away from the flow channel to form a first positioning point, and the first positioning points of the plurality of first positioning units are arranged at intervals along the circumferential direction. Two first positioning rods respectively belonging to two adjacent first positioning units converge at ends close to the flow channel to form a first node.

In the above embodiment, the first positioning part includes a plurality of first positioning units arranged along the circumferential direction. The plurality of first positioning units cooperate with one another to realize multi-point positioning of cardiac tissues in the circumferential direction. Each first positioning unit includes two first positioning rods connected at ends away from the flow channel to form a first positioning point. The first positioning points of various first positioning units realize multi-point positioning of corresponding positions of cardiac tissues, featuring a simple structure and high positioning stability and reducing the probability of displacement of the heart valve stent.

In some embodiments, the second positioning part includes a plurality of second positioning units. Each second positioning unit includes two second positioning rods, and ends of the two second positioning rods away from the flow channel converge to form a second positioning point. Ends of the two second positioning rods close to the flow channel are connected to two adjacent first nodes, wherein the first positioning point and the second positioning point are oppositely arranged along the axial direction of the flow channel and spaced apart along the axial direction of the flow channel.

In the above embodiment, a second positioning unit is disposed between two adjacent first positioning units. Ends of the second positioning rods of the second positioning unit away from the flow channel are connected to form a second positioning point, such that positioning is realized by abutting the second positioning point against the ventricular side of cardiac tissues.

In some embodiments, each first positioning rod includes a first axial segment and a first circumferential segment. One end of the first axial segment is connected to the first node, and another end of the first axial segment is connected to the first circumferential segment. In the first positioning part, a sum of circumferential extension angles of the first circumferential segments of each first positioning unit is defined as a first extension angle. Each second positioning rod includes a second axial segment and a second circumferential segment. One end of the second axial segment is connected to the first node, and another end of the second axial segment is connected to the second circumferential segment. In the second positioning part, a sum of circumferential extension angles of the second circumferential segments of each second positioning unit is defined as a second extension angle. The first extension angle is less than or equal to the second extension angle.

In the above embodiment, the first positioning rod of each first positioning unit abuts against one side of cardiac tissues via the first circumferential segment, which is designed in a circular arc shape, achieving reliable positioning and applying low pressure on cardiac tissues to reduce damage to cardiac tissues. Similarly, the second positioning rod of each second positioning unit abuts against the ventricular side of cardiac tissues via the second circumferential segment, which is designed in a circular arc shape, minimizing damage to the ventricular side of cardiac tissues.

In some embodiments, the first circumferential segment and the second circumferential segment are designed in a circular arc shape.

In some embodiments, the stent body includes a plurality of support units arranged along a circumferential direction to enclose the flow channel. Each support unit includes a first support rod and a second support rod configured to be connected with different artificial leaflets respectively. In the same support unit, an upstream end of the first support rod and an upstream end of the second support rod are respectively connected to two adjacent first nodes, and a downstream end of the first support rod and a downstream end of the second support rod are connected to form a second node.

In the above embodiment, the plurality of support units function as a framework of the stent body, and middle portions of the plurality of support units enclose the flow channel for blood circulation. Each support unit includes a first support rod and a second support rod respectively configured to connect different leaflets. Upstream ends of the first support rod and the second support rod are respectively connected to two first nodes, so that the plurality of support units are integrally connected with the first positioning part and the second positioning part.

In some embodiments, each support unit further includes a third support rod and a fourth support rod. One end of the third support rod and one end of the fourth support rod are connected to the same second node, and another end of the third support rod and another end of the fourth support rod are respectively connected to two adjacent first nodes. A connecting ring is provided at a most downstream end of each of the third support rod and the fourth support rod.

In the above embodiment, one end of the third support rod and one end of the fourth support rod are connected to the same second node, and another end of the third support rod and another end of the fourth support rod are respectively connected to two adjacent first nodes, so that structural stability of the stent body is improved. A connecting ring is formed at the most downstream end of each of the third support rod and the fourth support rod, that is, each support unit is provided with two connecting rings, and then the plurality of support units enable the stent body to be provided with a plurality of connecting rings for connection with a delivery system with high connection stability.

In some embodiments, a first rivet member is disposed at each first node. In the first rivet member, an end of the first positioning rod close to the flow channel is connected to the first support rod or the second support rod, and an end of the second positioning rod close to the flow channel is connected to the third support rod or the fourth support rod.

In the above embodiment, the end of the first positioning rod close to the flow channel is connected to the first support rod or the second support rod, and the end of the second positioning rod close to the flow channel is connected to the third support rod or the fourth support rod. The first rivet member can integrally connect intersection portions of the first positioning part, the second positioning part and the support unit at the first node to ensure integrity of the heart valve stent.

In some embodiments, a second rivet member is disposed at each second node. In the second rivet member, the first support rod is connected to the third support rod, and the second support rod is connected to the fourth support rod.

In the above embodiment, the first support rod is connected to the third support rod, and the second support rod is connected to the fourth support rod. The second rivet member can integrally connect the first support rod, the second support rod, the third support rod and the fourth support rod to improve integrity of the support unit, thereby effectively guaranteeing connection reliability of each support rod in each support unit.

In some embodiments, the heart valve stent is an aortic valve stent. The first positioning part is configured to be positioned at aortic sinuses, and the second positioning part is configured to be positioned at a ventricular side of an aortic valve.

In some embodiments, the heart valve stent is woven from at least one filament member.

In the above embodiment, the heart valve stent is woven from at least one filament member, which facilitates deformation for delivery via a delivery system. When delivered to a native aortic valve, the filament member can quickly recover its original shape and improve reliability of supporting the heart valve stent at the aortic valve.

In some embodiments, when the heart valve stent is woven from a plurality of filament members, two connected filament members are fixedly connected via riveting through a riveting structure or welding.

In some embodiments, a material of the filament member is a memory alloy wire or a nickel-titanium alloy wire.

In some embodiments, the first support rod, the second support rod, the third support rod or the fourth support rod is woven from a variable-diameter memory alloy wire.

In addition, embodiments of the present invention further provide a heart valve prosthesis. The heart valve prosthesis includes leaflets and the aforementioned heart valve stent, and the leaflets are disposed inside the flow channel of the stent body.

In some embodiments, the heart valve prosthesis includes a first skirt and a second skirt. The first skirt is connected and covers a space formed among the first support rods, the second support rods and the first positioning rods of the heart valve stent. The second skirt surrounds an outer peripheral side of the first skirt and is hermetically connected with the first skirt.

In some embodiments, materials of the first skirt and the second skirt are biological materials, polymers or woven fabrics.

In some embodiments, the second skirt is integrally in an outward protruding annular structure.

In the above embodiment, the leaflets are disposed inside the flow channel and connected with the heart valve stent. Opening and closing of the leaflets can control blood circulation inside the flow channel. For example, during cardiac systole, the leaflets open to deliver blood inside the heart to the whole body through the aorta. Meanwhile, during cardiac diastole, the leaflets close in a timely manner to prevent blood inside the aorta from flowing back to the ventricular side. Therefore, sealing performance of the flow channel inside the heart valve prosthesis is particularly critical. The first skirt surrounds a space region formed among the first support rods, the second support rods and the first positioning rods of the heart valve stent, thereby preventing blood from flowing through a peripheral side of the heart valve stent and ensuring that blood can only flow in from the upstream end of the flow channel and flow out from the downstream end of the flow channel. The second skirt hermetically connected with the first skirt further surrounds the outer peripheral side of the first skirt, such that the second skirt is configured to block blood regurgitation and avoid paravalvular leakage.

Other features and advantages of the present invention will be described in detail in the following part of "Detailed Description of the Embodiments".

### BRIEF DESCRIPTION OF THE DRAWINGS

For clearer illustration of embodiments of the present invention, the drawings required for describing the embodiments will be briefly introduced below. It should be understood that the following drawings merely illustrate some embodiments of the present invention and shall not be regarded as a limitation on the scope thereof. Those skilled in the art can derive other relevant drawings from these drawings without creative efforts.
FIG. 1 is a schematic structural view of a normal heart valve in an open state;
FIG. 2 is a schematic structural view of a normal heart valve in a closed state;
FIG. 3 is a schematic structural view of a diseased heart valve in an open state;
FIG. 4 is a schematic structural view of a diseased heart valve in a closed state;
FIG. 5 is a schematic structural view of a heart valve stent provided by some embodiments of the present invention;
FIG. 6 is a schematic structural view of the heart valve stent provided by some embodiments of the present invention from another angle;
FIG. 7 is a schematic structural view of a heart valve prosthesis provided by some embodiments of the present invention;
FIG. 8 is a schematic structural view of a second skirt shown in FIG. 7;
FIG. 9 is a schematic view illustrating positioning of the heart valve stent at an aortic valve according to some embodiments of the present invention;
FIG. 10 is an enlarged schematic view of a portion A shown in FIG. 9; and
FIG. 11 is a schematic view illustrating positioning of the heart valve prosthesis at an aortic valve according to some embodiments of the present invention.

Reference Numerals: 10 - stent body; 11 - support unit; 12 - first support rod; 13 - second support rod; 14 - third support rod; 15 - fourth support rod; 16 - connecting ring; 20 - first positioning part; 21 - first positioning unit; 210 - first positioning rod; 2101 - first axial segment; 2102 - first circumferential segment; 211 - first positioning point; 30 - second positioning part; 31 - second positioning unit; 310 - second positioning rod; 3101 - second axial segment; 3102 - second circumferential segment; 311 - second positioning point; 40 - first node; 41 - first rivet member; 50 - second node; 51 - second rivet member; 100 - heart valve stent; 200 - leaflet; 300 - first skirt; 400 - second skirt; 401 - blood filling port; 500 - aortic valve; 501 - native valve annulus; 502 - native leaflet; 503 - left ventricle; 504 - aorta; 1000 - heart valve prosthesis.

### DETAILED DESCRIPTION OF THE EMBODIMENTS

To make purposes, technical solutions and advantages of embodiments of the present invention clearer, the technical solutions of embodiments of the present invention will be clearly and completely described below with reference to the drawings of embodiments of the present invention. Apparently, the described embodiments are part of embodiments of the present invention, rather than all embodiments. Assemblies of embodiments of the present invention generally described and illustrated in the drawings can be arranged and designed in various different configurations.

Therefore, the following detailed description of embodiments of the present invention provided in the drawings is not intended to limit the scope of the claimed present invention, but merely represents selected embodiments of the present invention. All other embodiments obtained by those skilled in the art based on embodiments of the present invention without creative efforts shall fall within the protection scope of the present invention.

It should be noted that similar reference numerals and letters represent similar items in the following drawings. Therefore, once an item is defined in one drawing, no further definition or explanation thereof is required in subsequent drawings.

In descriptions of embodiments of the present invention, it shall be noted that orientation or positional relationships indicated are based on orientations or positional relationships shown in the drawings, or conventional placement orientations or positional relationships of products of the application when in use, merely for facilitating description of the present invention and simplifying descriptions, rather than indicating or implying that the indicated device or element must have a specific orientation and be constructed and operated in a specific orientation, and thus shall not be construed as a limitation on the present invention. In addition, terms such as "first", "second", "third" are merely used for distinguishing descriptions and shall not be construed as indicating or implying relative importance.

In descriptions of the present invention, it shall also be noted that unless otherwise clearly specified and limited, terms "arrange" and "connect" shall be understood in a broad sense. For example, the connection may be fixed connection, detachable connection or integral connection; the connection may be direct connection or indirect connection via an intermediate medium, or internal communication between two elements. Those skilled in the art can understand specific meanings of the above terms in the present invention based on the specific context.

### Embodiment

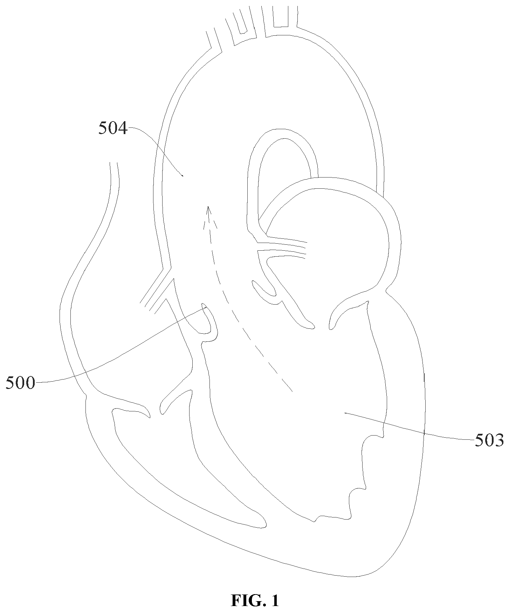

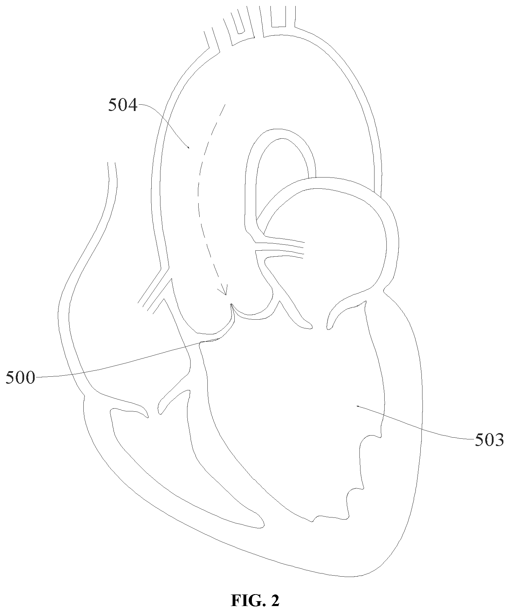

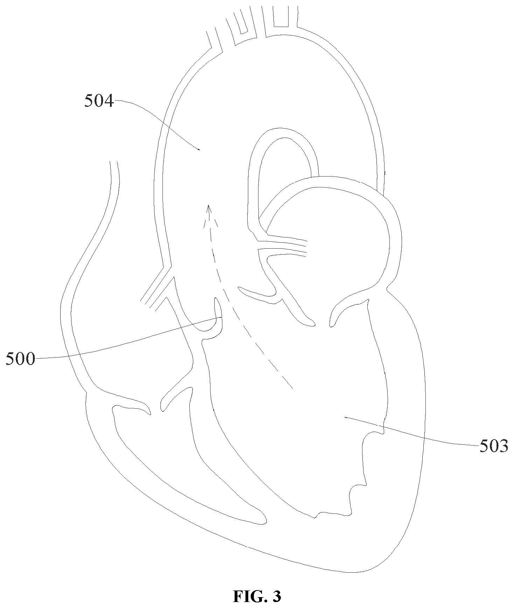

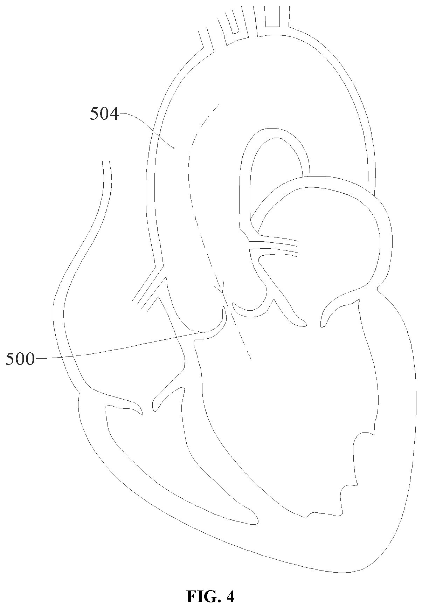

Heart valves grow between the atrium and the ventricle, and between the ventricle and the large artery, function as one-way valves and are configured to guide unidirectional blood flow. The four valves of the human body are respectively the mitral valve, tricuspid valve, valve of aorta 504 and pulmonary valve. If lesions (such as stenosis or insufficiency) occur on these valves, blood flow will be affected, thereby causing abnormal cardiac function and ultimately leading to heart failure. Specifically, FIG. 1 schematically illustrates a normal heart valve in an open state. A left ventricle 503 contracts, an aortic valve 500 opens, and blood flows from the left ventricle 503 to an aorta 504. FIG. 2 schematically illustrates a normal heart valve in a closed state. The left ventricle 503 relaxes, the aortic valve 500 closes, and blood cannot flow from the aorta 504 to the left ventricle 503. FIG. 3 schematically illustrates a diseased heart valve in an open state. The left ventricle 503 contracts, the aortic valve 500 opens incompletely, and blood flows from the left ventricle 503 to the aorta 504. FIG. 4 schematically illustrates a diseased heart valve in a closed state. The left ventricle 503 relaxes, the aortic valve 500 closes incompletely, and blood can flow from the aorta 504 to the left ventricle 503. Therefore, valve replacement surgery is mostly adopted for treatment when valves develop lesions, namely replacement with artificial mechanical valves or bioprosthetic valves.

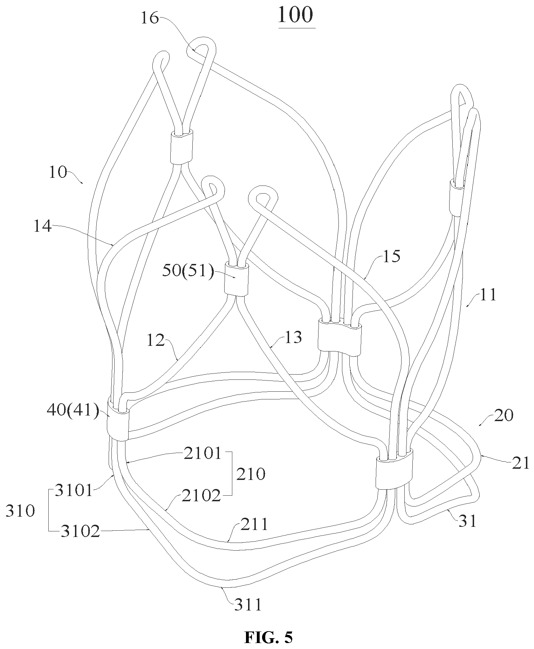

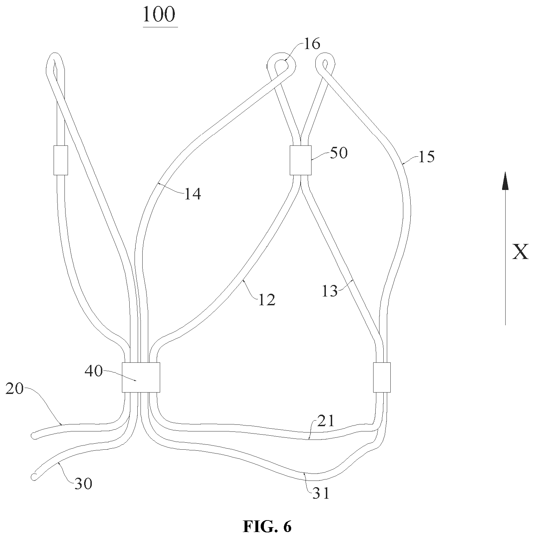

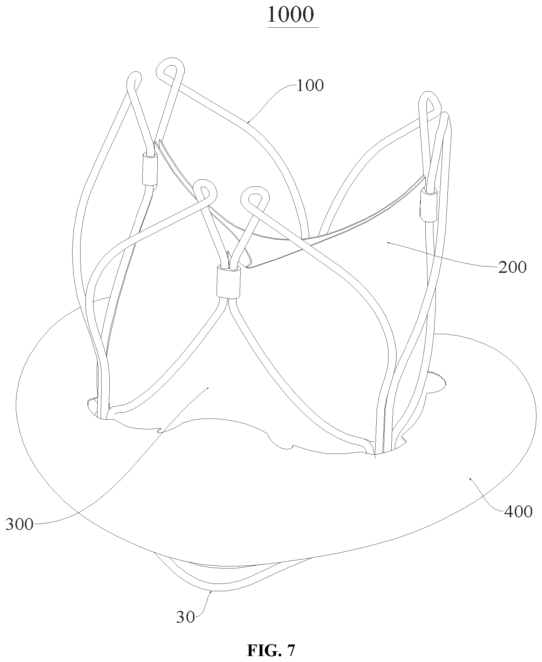

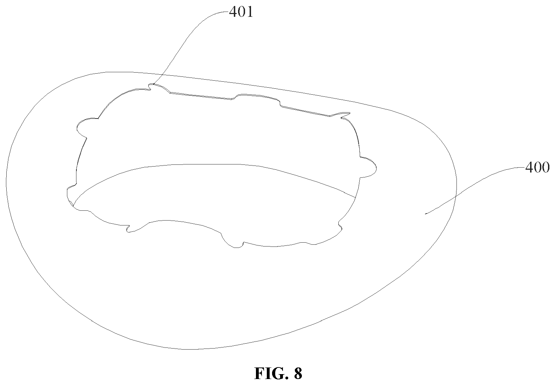

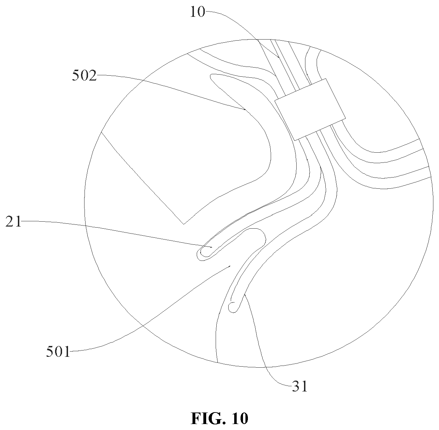

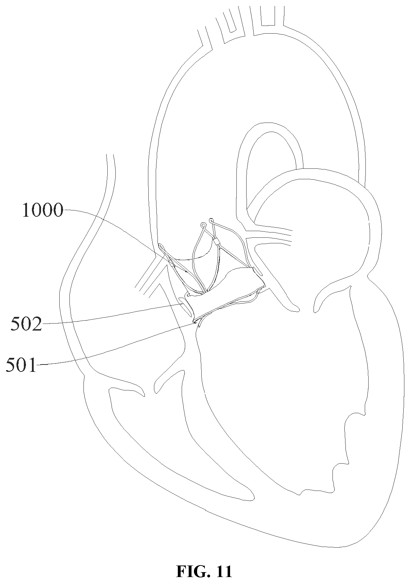

In view of the above, embodiments of the present invention provide a heart valve stent 100. Referring to FIG. 5 to FIG. 11, the heart valve stent 100 includes a stent body 10, and a first positioning part 20 and a second positioning part 30 configured to position relative to cardiac tissues. The stent body 10 defines a flow channel for blood circulation therein. An upstream direction and a downstream direction are defined according to a direction X in which blood flows through the flow channel. Both the first positioning part 20 and the second positioning part 30 are located at an upstream end of the flow channel, and both the first positioning part 20 and the second positioning part 30 extend radially outward from the stent body 10, wherein the second positioning part 30 is located on an upstream side of the first positioning part 20. Along a circumferential direction of the flow channel, the first positioning part 20 and the second positioning part 30 at least partially overlap. Along an axial direction of the flow channel, a spacing is provided between the first positioning part 20 and the second positioning part 30.

In the present technical solution, a flow channel for blood circulation is defined in a middle portion of the stent body 10 of the heart valve stent 100 to allow blood to flow therethrough. Both the first positioning part 20 and the second positioning part 30 of the heart valve stent 100 are disposed at the upstream end of the flow channel. The first positioning part 20 and the second positioning part 30 abut against and clamp two sides of corresponding cardiac tissues to fix the heart valve stent 100, preventing displacement of the heart valve stent 100 caused by pressure exerted by blood on leaflets 200 when the leaflets 200 are closed. Therefore, the stability and reliability of the heart valve stent 100 on cardiac tissues are improved, and the service life of the heart valve stent 100 is prolonged. Moreover, the second positioning part 30 is disposed on the upstream side of the first positioning part 20, that is, the second positioning part 30 abuts against a ventricular side of cardiac tissues, and the first positioning part abuts against the other side of the cardiac tissues. The first positioning part 20 and the second positioning part 30 at least partially overlap, and a spacing is provided between the first positioning part 20 and the second positioning part 30 along the axial direction of the flow channel, such that the first positioning part 20 and the second positioning part 30 respectively clamp two opposite sides of the cardiac tissues with strong anchoring stability. The second positioning part 30 has low rigidity and good compliance, and exerts little influence on conduction bundles.

Herein, the cardiac tissues may be the mitral valve, tricuspid valve, valve of aorta 504 and pulmonary valve. For example, in the present embodiment, when the cardiac tissue is the aortic valve 504, the first positioning part 20 abuts against native aortic sinuses, and the second positioning part 30 abuts against a ventricular side of the aortic valve 500.

In some embodiments, a circumferential extension angle of the first positioning part 20 or the second positioning part 30 is α, where α is greater than 320°.

In the present embodiment, the circumferential extension angles of the first positioning part 20 and the second positioning part 30 are substantially equal.

In some embodiments, a connecting ring 16 is disposed on the stent body 10, and the connecting ring 16 is configured to be connected with a delivery system. The connecting ring 16 is formed on the stent body 10 and connected with the delivery system, so that the heart valve stent 100 can be delivered to a target position of cardiac tissues via the delivery system, improving the stability and reliability during delivery of the heart valve stent 100. In addition, arrangement of the connecting ring 16 enables full retrieval of the heart valve stent 100.

In some embodiments, the first positioning part 20 includes a plurality of first positioning units 21 arranged along a circumferential direction. Each first positioning unit 21 includes two first positioning rods 210. Two first positioning rods 210 belonging to the same first positioning unit 21 are connected at ends away from the flow channel to form a first positioning point 211, and the first positioning points 211 of the plurality of first positioning units 21 are arranged at intervals along the circumferential direction. Two first positioning rods 210 respectively belonging to two adjacent first positioning units 21 converge at ends close to the flow channel to form a first node 40. The first positioning part 20 includes a plurality of first positioning units 21 arranged along the circumferential direction and the plurality of first positioning units 21 cooperate with one another to realize multi-point positioning of cardiac tissues in the circumferential direction. Each first positioning unit 21 includes two first positioning rods 210 connected at ends away from the flow channel to form a first positioning point 211. The first positioning points 211 of the first positioning units 21 realize multi-point positioning of corresponding positions of cardiac tissues, featuring a simple structure and high positioning stability and reducing the probability of displacement of the heart valve stent 100.

Herein, the number of the first positioning units 21 can be two, three, four or the like. In the present embodiment, the number of the first positioning units 21 is three.

In some embodiments, the second positioning part 30 includes a plurality of second positioning units 31. Each second positioning unit 31 includes two second positioning rods 310, and ends of the two second positioning rods 310 away from the flow channel converge to form a second positioning point 311. Ends of the two second positioning rods 310 close to the flow channel are connected to two adjacent first nodes 40, wherein the first positioning point 211 and the second positioning point 311 are oppositely arranged in the axial direction of the flow channel and spaced apart along the axial direction of the flow channel. A second positioning unit 31 is disposed between two adjacent first positioning units 21 and ends of the second positioning rods 310 of the second positioning unit 31 away from the flow channel are connected to form a second positioning point 311, such that positioning is realized by abutting the second positioning point 311 against the ventricular side of cardiac tissues.

In some embodiments, each first positioning rod 210 includes a first axial segment 2101 and a first circumferential segment 2102. One end of the first axial segment 2101 is connected to the first node 40, and the other end thereof is connected to the first circumferential segment 2102. In the first positioning part 20, a sum of circumferential extension angles of the first circumferential segments 2102 of each first positioning unit 21 is defined as a first extension angle. Each second positioning rod 310 includes a second axial segment 3101 and a second circumferential segment 3102. One end of the second axial segment 3101 is connected to the first node 40, and the other end thereof is connected to the second circumferential segment 3102. In the second positioning part 30, a sum of circumferential extension angles of the second circumferential segments 3102 of each second positioning unit 31 is defined as a second extension angle. The first extension angle is less than or equal to the second extension angle. The first positioning rod 210 of each first positioning unit 21 abuts against one side of cardiac tissues via the first circumferential segment 2102, which is designed in a circular arc shape, achieving reliable positioning and applying low pressure on cardiac tissues to reduce damage to cardiac tissues. Similarly, the second positioning rod 310 of each second positioning unit 31 abuts against the ventricular side of cardiac tissues via the second circumferential segment 3102, which is designed in a circular arc shape, minimizing damage to the ventricular side of cardiac tissues.

In some embodiments, the stent body 10 includes a plurality of support units 11 arranged along a circumferential direction to enclose the flow channel. Each support unit 11 includes a first support rod 12 and a second support rod 13 configured to be connected with different artificial leaflets 200 respectively. In the same support unit 11, an upstream end of the first support rod 12 and an upstream end of the second support rod 13 are respectively connected to two adjacent first nodes 40, and a downstream end of the first support rod 12 and a downstream end of the second support rod 13 are connected to form a second node 50. The plurality of support units 11 function as a framework of the stent body 10, and middle portions of the plurality of support units 11 enclose the flow channel for blood circulation. Each support unit 11 includes a first support rod 12 and a second support rod 13 respectively configured to connect different leaflets 200 and upstream ends of the first support rod 12 and the second support rod 13 are respectively connected to two first nodes 40, so that the plurality of support units 11 are integrally connected with the first positioning part 20 and the second positioning part 30.

In some embodiments, each support unit 11 further includes a third support rod 14 and a fourth support rod 15. One end of the third support rod 14 and one end of the fourth support rod 15 are connected to the same second node 50, and the other ends thereof are respectively connected to two adjacent first nodes 40. A connecting ring 16 is formed at a most downstream end of each of the third support rod 14 and the fourth support rod 15. One end of the third support rod 14 and one end of the fourth support rod 15 are connected to the same second node 50, and the other ends thereof are respectively connected to two adjacent first nodes 40, so that structural stability of the stent body 10 is improved. A connecting ring 16 is formed at the most downstream end of each of the third support rod 14 and the fourth support rod 15, that is, each support unit 11 is provided with two connecting rings 16, and the plurality of support units 11 enable the stent body 10 to be provided with a plurality of connecting rings 16 for connection with a delivery system with high connection stability.

In some embodiments, a first rivet member 41 is disposed at each first node 40. In the first rivet member 41, an end of the first positioning rod 210 close to the flow channel is connected to the first support rod 12 or the second support rod 13, and an end of the second positioning rod 310 close to the flow channel is connected to the third support rod 14 or the fourth support rod 15. The end of the first positioning rod 210 close to the flow channel is connected to the first support rod 12 or the second support rod 13, and the end of the second positioning rod 310 close to the flow channel is connected to the third support rod 14 or the fourth support rod 15, such that the first rivet member 41 can integrally connect intersection portions of the first positioning part 20, the second positioning part 30 and the support unit 11 at the first node 40 to ensure integrity of the heart valve stent 100.

Herein, in the first rivet member 41, the first positioning rod 210 is connected to the first support rod 12 or the second support rod 13, and the second positioning rod 310 is connected to the third support rod 14 or the fourth support rod 15. Each rod member is arranged in parallel, which helps improve connection reliability of the first rivet member 41, improve product appearance, and facilitate contraction and expansion of the heart valve stent 100.

In some embodiments, a second rivet member 51 is disposed at each second node 50. In the second rivet member 51, the first support rod 12 is connected to the third support rod 14, and the second support rod 13 is connected to the fourth support rod 15. The first support rod 12 is connected to the third support rod 14, and the second support rod 13 is connected to the fourth support rod 15, such that the second rivet member 51 can integrally connect the first support rod 12, the second support rod 13, the third support rod 14 and the fourth support rod 15 to improve integrity of the support unit 11, thereby effectively guaranteeing connection reliability of each support rod in each support unit 11.

In some embodiments, the heart valve stent 100 is an aortic valve stent. The first positioning part 20 is configured to be positioned at aortic sinuses, and the second positioning part 30 is configured to be positioned at a ventricular side of an aortic valve 500.

In some embodiments, the heart valve stent 100 is woven from at least one filament member. The heart valve stent 100 is woven from at least one filament member, which facilitates deformation for delivery via a delivery system. When delivered to a native aortic valve 500, the filament member can quickly recover its original shape and improve reliability of supporting the heart valve stent 100 at the aortic valve 500.

Herein, the heart valve stent 100 may be woven from one filament member or a plurality of filament members. When the heart valve stent 100 is woven from a plurality of filament members, two connected filament members are fixedly connected via riveting through a riveting structure or welding. Of course, the heart valve stent 100 may also be fixedly connected through welding or threaded connection.

In addition, a material of the filament member may be a memory alloy wire, a nickel-titanium alloy wire or the like. The heart valve stent 100 is woven from memory alloy wires or expanded and formed from memory alloy meshes, and connection portions of memory alloy wires are connected via welding, riveting, bonding or the like.

Of course, each support rod in the support unit 11 may be made of a variable-diameter wire, that is, the first support rod 12, the second support rod 13, the third support rod 14 or the fourth support rod 15 is woven from a variable-diameter memory alloy wire, or a memory alloy tube may be partially sleeved on an outer peripheral side of the first support rod 12, the second support rod 13, the third support rod 14 or the fourth support rod 15 to partially increase the diameter of each support rod, thereby improving supporting force of the heart valve stent 100 and further improving supporting stability.

Embodiments of the present invention provide a heart valve prosthesis 1000. The heart valve prosthesis 1000 includes leaflets 200 and the aforementioned heart valve stent 100, and the leaflets 200 are disposed inside the flow channel of the stent body 10.

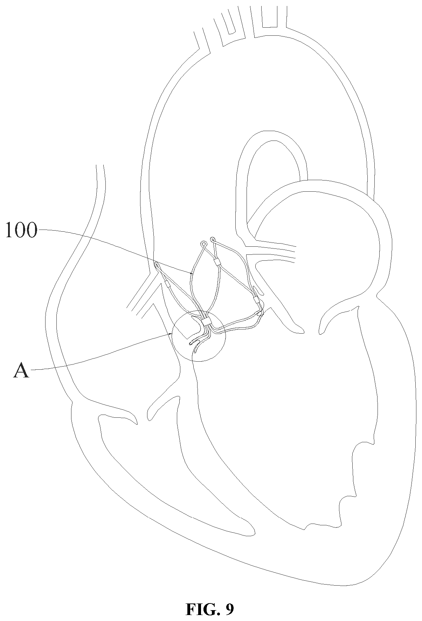

Referring to FIG. 9 to FIG. 11, after implantation of the heart valve stent 100 or the heart valve prosthesis 1000, the heart valve stent 100 or the heart valve prosthesis 1000 expands native leaflets 502 outward, and the leaflets 200 of the heart valve prosthesis 1000 replace the native leaflets 502. The first positioning part 20 and the second positioning part 30 of the heart valve prosthesis 1000 abut against two sides of a native valve annulus 501.

Herein, a material of the leaflets 200 may be one of polymer materials, biological tissue materials and tissue engineering materials. Specifically, the material of the leaflets 200 may be bovine pericardium, porcine pericardium, bovine or porcine heart valves, polymer materials or the like. In the present embodiment, a material of the leaflets 200 is a polymer material. In addition, a connection manner between the leaflets 200 and the heart valve stent 100 may be one of bonding, hot melting and polymer attachment. In the present embodiment, the leaflets 200 are coated on a surface of the heart valve stent 100 via polymer attachment with higher adhesion, thereby avoiding damage or shedding of the leaflets 200 caused by excessive stress during suture sewing, improving reliability of the leaflets 200 and helping prolong service life of the product.

In some embodiments, the heart valve prosthesis 1000 includes a first skirt 300 and a second skirt 400. The first skirt 300 is connected and covers a space formed among the first support rods 12, the second support rods 13 and the first positioning rods 210 of the heart valve stent 100. The second skirt 400 surrounds an outer peripheral side of the first skirt 300 and is hermetically connected with the first skirt 300.

In the above embodiment, the leaflets 200 are disposed inside the flow channel and connected with the heart valve stent 100. Opening and closing of the leaflets 200 can control blood circulation inside the flow channel. For example, during cardiac systole, the leaflets 200 open to deliver blood inside the heart to the whole body through the aorta 504. Meanwhile, during cardiac diastole, the leaflets 200 close in a timely manner to prevent blood inside the aorta 504 from flowing back to the ventricular side. Therefore, sealing performance of the flow channel inside the heart valve prosthesis 1000 is particularly critical. The first skirt 300 surrounds a space region formed among the first support rods 12, the second support rods 13 and the first positioning rods 210 of the heart valve stent 100, preventing blood from flowing through a peripheral side of the heart valve stent 100 and ensuring that blood can only flow in from the upstream end of the flow channel and flow out from the downstream end of the flow channel. The second skirt 400 hermetically connected with the first skirt 300 further surrounds the outer peripheral side of the first skirt 300, and the second skirt 400 is configured to block blood regurgitation and avoid paravalvular leakage.

Herein, materials of the first skirt 300 and the second skirt 400 may be biological materials, polymers or woven fabrics. The second skirt 400 is disposed on the outer peripheral side of the first skirt 300 and integrally forms an outward protruding annular structure. A circumferential portion of the second skirt 400 is folded toward a downstream direction of the heart valve stent 100 to form a flanged edge. When the leaflets 200 are closed, blood can only flow to an upper side of the second skirt 400 through the leaflets 200 and circulate above the second skirt 400, thereby effectively preventing blood regurgitation and avoiding paravalvular leakage. A blood filling port 401 is formed at an upper edge of the second skirt 400, and blood fills the port to form thrombus for preventing paravalvular leakage.

It should be noted that features of embodiments of the present invention may be combined with one another without conflict.

The above descriptions merely illustrate optional embodiments of the present invention and are not intended to limit the present invention. Various modifications and variations can be made to the present invention by those skilled in the art. Any modification, equivalent replacement, improvement or the like made within the spirit and principle of the present invention shall fall within the protection scope of the present invention.

### INDUSTRIAL APPLICABILITY

The present invention provides a heart valve stent and a heart valve prosthesis with high mounting stability and strong anchoring performance for heart valves.

In addition, it can be understood that the heart valve stent and the heart valve prosthesis of the present invention are reproducible and can be widely applied in the field of medical technology.

## Claims

1. A heart valve stent, **characterized by** comprising a stent body, and a first positioning part and a second positioning part configured to position relative to cardiac tissues, wherein the stent body defines a flow channel for blood circulation therein, an upstream direction and a downstream direction are defined according to a direction in which blood flows through the flow channel, both the first positioning part and the second positioning part are located at an upstream end of the flow channel, and both the first positioning part and the second positioning part extend radially outward from the stent body, wherein the second positioning part is located on an upstream side of the first positioning part, along a circumferential direction of the flow channel, the first positioning part and the second positioning part at least partially overlap, and along an axial direction of the flow channel, a spacing is provided between the first positioning part and the second positioning part.

2. The heart valve stent according to claim 1, wherein a circumferential extension angle of the first positioning part or the second positioning part is α, and wherein α is greater than 320°.

3. The heart valve stent according to claim 1, wherein a connecting ring is disposed on the stent body, and the connecting ring is configured to be connected with a delivery system.

4. The heart valve stent according to claim 1, wherein the first positioning part comprises a plurality of first positioning units arranged along a circumferential direction, and each first positioning unit comprises two first positioning rods;
two first positioning rods belonging to a same first positioning unit are connected at ends away from the flow channel to form a first positioning point, and the first positioning points of the plurality of first positioning units are arranged at intervals along the circumferential direction;
two first positioning rods respectively belonging to two adjacent first positioning units converge at ends close to the flow channel to form a first node.

5. The heart valve stent according to claim 4, wherein the second positioning part comprises a plurality of second positioning units, each second positioning unit comprises two second positioning rods, ends of the two second positioning rods away from the flow channel converge to form a second positioning point; and ends of the two second positioning rods close to the flow channel are connected to two adjacent first nodes,
wherein the first positioning point and the second positioning point are oppositely arranged along the axial direction of the flow channel and spaced apart along the axial direction of the flow channel.

6. The heart valve stent according to claim 5, wherein each first positioning rod comprises a first axial segment and a first circumferential segment, one end of the first axial segment is connected to the first node, and another end of the first axial segment is connected to the first circumferential segment; and in the first positioning part, a sum of circumferential extension angles of the first circumferential segments of each first positioning unit is defined as a first extension angle; and
each second positioning rod comprises a second axial segment and a second circumferential segment, one end of the second axial segment is connected to the first node, and another end of the second axial segment is connected to the second circumferential segment; in the second positioning part, a sum of circumferential extension angles of the second circumferential segments of each second positioning unit is defined as a second extension angle; and the first extension angle is less than or equal to the second extension angle.

7. The heart valve stent according to claim 6, wherein the first circumferential segment and the second circumferential segment are designed in a circular arc shape.

8. The heart valve stent according to claim 5, wherein the stent body comprises a plurality of support units arranged along the circumferential direction to enclose the flow channel; and
each support unit comprises a first support rod and a second support rod configured to be connected with different artificial leaflets respectively; and in the same support unit, an upstream end of the first support rod and an upstream end of the second support rod are respectively connected to two adjacent first nodes, and a downstream end of the first support rod and a downstream end of the second support rod are connected to form a second node.

9. The heart valve stent according to claim 8, wherein each support unit further comprises a third support rod and a fourth support rod;
one end of the third support rod and one end of the fourth support rod are connected to a same second node, and another end of the third support rod and another end of the fourth support rod are respectively connected to two adjacent first nodes; and
a connecting ring is provided at a most downstream end of each of the third support rod and the fourth support rod.

10. The heart valve stent according to claim 9, wherein a first rivet member is disposed at each first node; and in the first rivet member, an end of the first positioning rod close to the flow channel is connected to the first support rod or the second support rod, and an end of the second positioning rod close to the flow channel is connected to the third support rod or the fourth support rod.

11. The heart valve stent according to claim 9, wherein a second rivet member is disposed at each second node; and in the second rivet member, the first support rod is connected to the third support rod, and the second support rod is connected to the fourth support rod.

12. The heart valve stent according to any one of claims 1 to 11, wherein the heart valve stent is an aortic valve stent, the first positioning part is configured to be positioned at aortic sinuses, and the second positioning part is configured to be positioned at a ventricular side of an aortic valve.

13. The heart valve stent according to any one of claims 1 to 11, wherein the heart valve stent is woven from at least one filament member.

14. The heart valve stent according to claim 13, wherein when the heart valve stent is woven from a plurality of filament members, two connected filament members are fixedly connected via riveting through a riveting structure or welding.

15. The heart valve stent according to claim 13 or 14, wherein a material of the filament member is a memory alloy wire or a nickel-titanium alloy wire.

16. The heart valve stent according to any one of claims 8 to 11, wherein the first support rod, the second support rod, the third support rod or the fourth support rod is woven from a variable-diameter memory alloy wire.

17. A heart valve prosthesis, **characterized by** comprising leaflets and the heart valve stent according to any one of claims 1 to 16, wherein the leaflets are disposed inside the flow channel of the stent body.

18. The heart valve prosthesis according to claim 17, comprising:
a first skirt, connected and covering a space formed among the first support rods, the second support rods and the first positioning rods of the heart valve stent;
a second skirt, surrounding an outer peripheral side of the first skirt and hermetically connected with the first skirt.

19. The heart valve prosthesis according to claim 18, wherein materials of the first skirt and the second skirt are biological materials, polymers or woven fabrics.

20. The heart valve prosthesis according to claim 18 or 19, wherein the second skirt is integrally in an outward protruding annular structure.
